# EUROPEAN PATENT APPLICATION

(11) **EP 1 790 410 A1**
(43) Date of publication of application: **30.05.2007**
(21) Application number: 05405669.2
(22) Date of filing: 25.11.2005
(51) Int. Cl.: B01D 53/88, B01J 19/12, B01J 19/24

(54) **Apparatus for photocatalytically cleaning and deodorizing fluids**

(71) Applicant: BUXAIR N.V., Curacao (AN)
(72) Inventor: Pastor Jean-Pierre, Caracas (VE)
(74) Representative: Reuteler, Raymond Werner

(57) **Abstract**

A catalyser device for photo-catalytically cleaning and deodorizing air comprises an air conduit having a tubular wall portion and a plurality of spaced apart blades extending from an inner surface of the tubular portion towards a central axis of the conduit.

## Description

The present invention relates to an apparatus for cleaning and deodorizing fluids, in particular air.

There are numerous air purification systems that have been described, or that are commercially available, aiming to remove various pollutants, such as dust, micro-particles, noxious gases, allergens, and pathogenic micro-organisms from the air. Dust and other micro-particles are removed from the air by use of filters, and it is known to remove certain volatile and reactive molecules by the use of catalytic processes. Micro-organisms may be destroyed by ultraviolet light radiation. It is known to combine the use of ultraviolet light radiation and photocatalytic processes to accelerate the degradation of noxious particles and the destruction of micro-organisms.

The use of photo-catalysts in devices for deodorizing or purifying air is described in a number of patent publications, for example, US 5,670,126, US 6,558,639, US 2,002,094,298, US 6,358,374, US 2,004,007,453, JP 11226357, WO 02/085989, and FR 2821558. The most common photo-catalyst used in known systems is titanium dioxide because of its excellent photocatalytic activity when irradiated with ultraviolet radiation and its harmlessness to the human organism.

In known systems the photo-catalyst is generally combined with a binder to form a photocatalytic coating material that is coated on a filter element or other surfaces of the air purifying system irradiated by an ultraviolet light source, typically an ultraviolet lamp. For example EP 0978690 and WO 96/37281 describe systems in which air is passed through an air-permeable sheet supporting a photocatalytic material and placed in the vicinity of a UV light source.

There are a number of drawbacks with the known systems. For instance the known air purifying and deodorising systems are generally relatively costly to manufacture and maintain.

Also the known systems do not have optimal air cleaning properties with respect to removing certain air-born polluants, allergens and pathogens. Particularly in respect of certain pathogenic micro-organisms such as bacteria, protozoa, spores.

In view of the aforegoing, it is an object of this invention to provide a versatile, economic, reliable and effective catalyser device for use in the photocatalytic treatment of fluids, in particular air.

It would be advantageous to provide an apparatus incorporating a catalyser device for purification of air, that is economic to manufacture, versatile, reliable, and effective.

It would be advantageous to provide an apparatus that is easy and economical to use and maintain.

Objects of this invention have been achieved by providing a catalyser device according to claim 1.

Disclosed herein is a catalyser device for cleaning and deodorising air, that includes an air conduit and a photocatalytic material in the conduit. The air conduit has a tubular wall portion and a plurality of spaced apart blades extending therefrom towards the central zone of the conduit, in which an ultra-violet light source is mountable.

Advantageously the inner surface of the tubular wall portion and the blades are coated with the photocatalytic material.

Advantageously the introduction of a tubular air conduit with presence of a number of spaced blades provides an effective and efficient treatment of air passing through the device. The blades not only increase the flow-path and surface area of photocatalytic material in contact with the air, but also create some turbulence to ensure that the air is well mixed and comes into contact with the photo-catalytic material. This is particularly advantageous for the destruction of spores and other micro organisms or volatile compounds that are not easily destroyed or decomposed by the ultraviolet light alone.

One or more of the blades may advantageously be inclined relative to the central axis of the conduit or relative to the inner surface of the tubular wall portion. Preferably the blades are inclined relative to the central axis of the conduit or relative to the inner surface of the tubular wall portion.

The angle of incline of the blades relative to the central axis may be between 0° to 90°, preferably between 45° to 85°, for instance from 55° to 75°. The angle of incline of the blades relative to the inner surface of the tubular wall portion may be between 40 to 140°, preferably between 55° to 80°, for instance around 60°.

Advantageously having one or more blades inclined relative to the central axis of the conduit and/or relative to the inner surface of the tubular wall portion enables optimal exposure of the surface of the blades coated with photocatalytic material to UV light irradiation from a UV light source placed in the conduit. The inclined blades also play a role in optimising the contact of air flowing through the device with the photocatalytic material.

According to a particular feature of the invention the tubular wall portion is constructed from a plurality of releasably connected annular elements, which may each comprise one or more blades. The annular elements can easily be manufactured independently and assembled to form the tubular portion. This feature enables an easy and economic construction and maintenance of the catalyser device.

The catalyser device may advantageously be made of a low cost polymer-based material that could be made in a tube shape for example by integrally injection moulding the tubular portion and blades, for example as annular stackable elements. It would also be possible to form the tubular portion and blades separately and then assemble them together by binding, welding or other fixing means.

Alternatively the tubular portion may be made from a sheet that is folded into a tube with the desired profile and fixed along the seam thereof. The sheet may be made from a polymer or metal material, or from a woven fibre such as woven fibreglass. Woven fibreglass is resistant to UV light and the catalytic action of the photocatalytic semiconductor, presents a very good support for adhesion of the coating, and is economic to manufacture.

The catalyser device according to this invention may thus be easily and economically replaced at regular intervals, for example annually. The ability to easily and economically change the catalyser device enables the apparatus to have a long operation lifetime, while at the same time ensuring effective deodorizing and cleaning properties in a very economic manner. The ability to use common light-weight materials for the catalyser device allows the apparatus to be light and versatile, and to be implemented in portable or fixed apparatuses for domestic or industrial use.

The photocatalytic material may be any known photocatalytic material. Advantageously the photocatalytic material may comprise particles of a semi-conductor, for instance cadmium sulfide, zinc sulfide and/or titanium dioxide in anatase cristalline form, which all exhibit high photocatalysis effects. Preferably, the photocatalytic material comprises titanium dioxide because of its effective catalytic activity in the presence of ultraviolet light, in addition to its stability and harmlessness to the human organism.

In a particular embodiment of the present invention the photocatalytic material comprises an inorganic binder including ultraviolet light permeable polymeric molecules, a polar diluent, and particles of an inorganic semi-conductor. Advantageously, the weight of the inorganic semi-conductor particles is two to twenty times less than the weight of both the diluent and binder. The polymeric molecules may include acrylic molecules. For example a photocatalytic gel as described in PCT/IB04/02417 may be used.

Objects of this invention have also been achieved by providing an apparatus for cleaning and deodorizing air according to claim 12.

Disclosed herein is an apparatus for cleaning and deodorizing air, comprising a housing, a catalyser device comprising an air conduit and a photocatalytic material in the conduit, and an ultraviolet light source received in the air conduit for irradiating an inner surface thereof, wherein said air conduit and comprises a tubular wall portion and a plurality of blades extending from an inner surface of the tubular wall portion towards a central region of the conduit. The catalyser device may advantageously be removably mounted in the housing. The apparatus may further comprise an airflow system for propelling or drawing air through the catalyser device,

The catalyser device may advantageously comprise any or all of the further characteristics described hereinabove.

Advantageously, the tubular wall portion of the catalyser device can be provided with a relatively large surface in comparison to the ultraviolet light source and with a reasonable length to ensure effective contact with the air flowing therethrough. The distance between the inner irradiated surface of the tubular portion is preferably between 1 cm to 3cm from the ultraviolet light source in order to optimize the overall effectiveness of the cleaning and deodorizing activity of the apparatus by optimizing the balance between the intensity of the photocatalytic activity of the gel and the direct germicidal action of the UV light with the surface area of the photocatalytic material in contact with the air.

Advantageously a radially interior edge of the plurality of blades extending from the inner surface of the tubular portion may have a form complementary with the contour of an outside surface of the ultraviolet light source, in order to optimise the contact of the air flowing through the air conduit with the blades and inner surface of the tubular wall portion.

The tubular wall portion may be provided with a generally cylindrical shape, or with other profiles such as elliptical or polygonal, depending on the arrangement of the ultraviolet light source and the projection of light rays therefrom.

Advantageously the present invention as described above provides for efficient cleaning and deodorising of air, including with respect to the destruction of micro organisms such as bacteria, protozoa and spores.

A more complete appreciation of the present invention and many of its advantages, aims and characteristics will be further understood from the claims and the following detailed description, in connection with the accompanying drawings, in which:
Figure 1 is a perspective exploded view of an apparatus with catalyser device for cleaning and deodorising air according to an embodiment of the present invention;
Figure 2A is a cut-out perspective view of an apparatus with catalyser device for cleaning and deodorising air according to an embodiment of the present invention;
Figure 2B is a cut-out perspective view of a catalyser device of the apparatus shown in figure 2A;
Figure 3 is a cut-out perspective view of an annular element of a catalyser device according to an embodiment of the invention.
Figures 4A to 4D are perspective views of annular elements of a catalyser device according to an embodiment of the present invention.

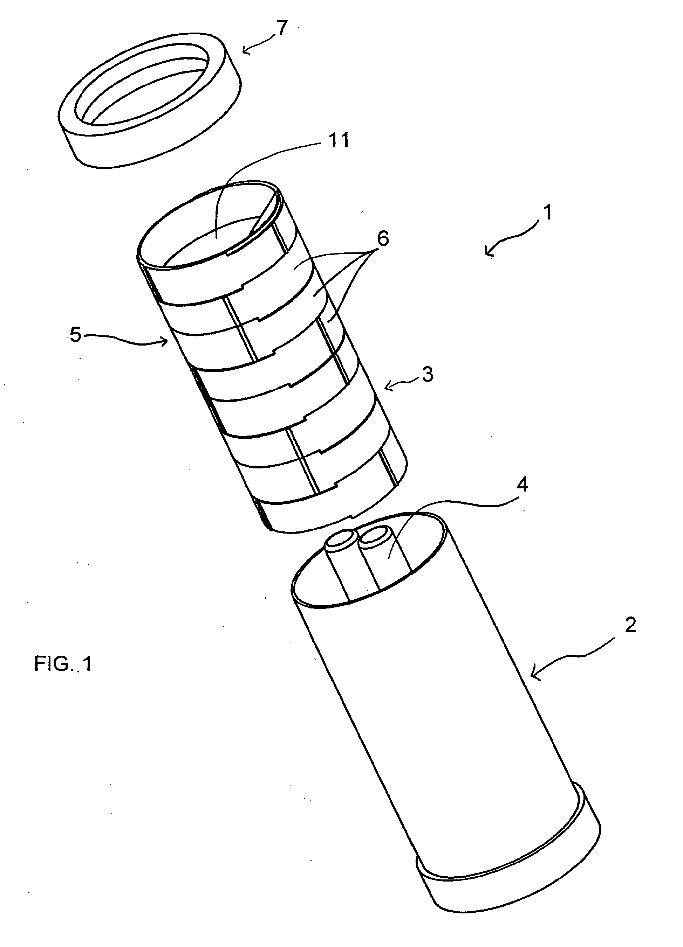

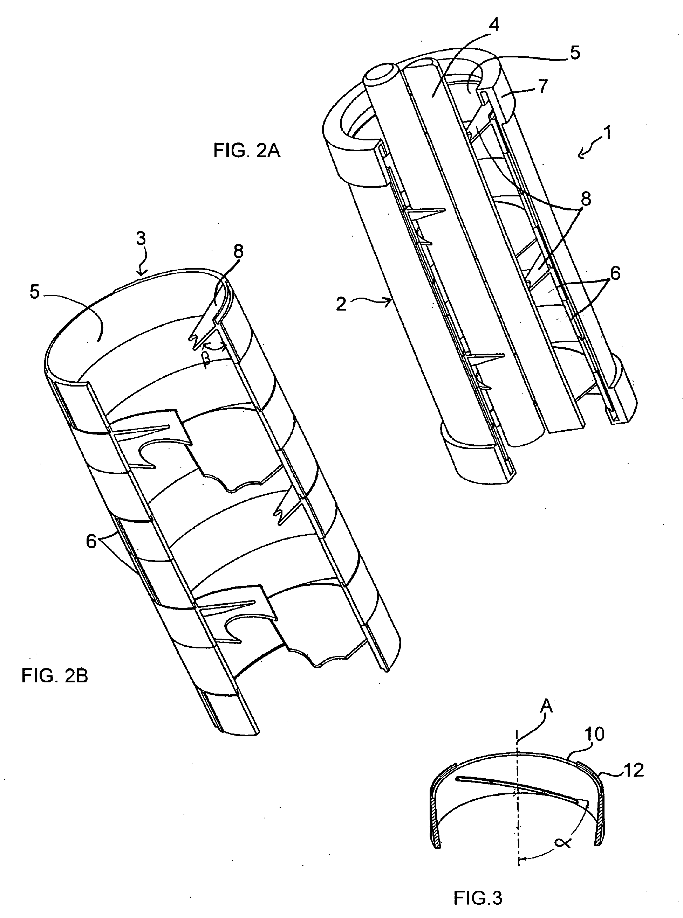

Referring to the figures, in particular figures 1 and 2A, an apparatus 1 for cleaning and deodorising air according to the present invention includes a housing 2, a catalyser device 3 removably mountable in the housing and an ultraviolet light source 4 which is received in the catalyser device. The catalyser device has a tubular wall portion 5, forming an air conduit 11, and a plurality of spaced apart blades 8 extending from the inner surface 5 of the tubular wall portion to a central region of he air conduit.

The plurality of spaced apart blades are preferably positioned around the circumference of the tubular portion. Advantageously the blades may be positioned in a generally helical arrangement as illustrated in figure 2B. An approximately helical arrangement of the blades provides for particularly advantageous flow properties of air passed through the catalyser devise, providing effective contact of the air with the inner surface of the tubular portion and the blades coated with photocatalytic material.

The illustrated embodiment further includes an annular housing element 7, which may be used to releasably hold the catalyser device in the housing.

The tubular wall portion and blades may advantageously be made of a polymeric material, for example as a rigid injection moulded plastic element, or flexible or thermoformable sheet of material folded, and if needed bonded along a seam to form a tube. In the latter embodiment (not shown), the blades could be integrally formed with the sheet by stamping and forming them out of the base sheet material, or by moulding them with the base sheet material in the case of a moulded sheet. Alternatively, the blades could be made separately and bonded, welded or otherwise fixed to the sheet.

In a preferred embodiment the catalyser device is formed from a plurality of annular elements 6, that may advantageously be formed by moulding of a polymer material such as a plastic. The blade portions may advantageously be formed integrally with the tubular wall portion.

The polymer may be polyethylene, polybutadieneterephtalate, polybutadienestyreneacrylonitrile, polymethyl methacrylate, polyvinylchloride or fibre-glass mineral or cellulosic types of polymers. The tubular wall portion and blades may also be made of other cost effective materials such as sheet metal bent into a tube and welded, crimped or bonded along its seam.

At least an inner surface of the tubular wall portion 5 and the blades 8, which are irradiated with ultraviolet light from the ultraviolet light source, are coated with a photocatalytic material. The photocatalytic material may be any known photocatalytic material. Preferably the photocatalytic material may comprise particles of a semi-conductor, for instance cadmium sufilde, zinc sulfide and/or titanium dioxide in anatase cristalline form, which all exhibit high photocatalysis effects. More preferably, the photocatalytic material comprises titanium dioxide because of its effective catalytic activity in the presence of ultraviolet light, in addition to its stability and harmlessness to the human organism.

In a particular embodiment of the present invention the photocatalytic material comprises an inorganic binder including ultraviolet light permeable polymeric molecules, a polar diluent, and particles of an inorganic semi-conductor. The polymeric molecules preferably include acrylic molecules, for example a as described in PCT/IB04/02417. Such a photocatalytic material exhibits good adhesion to many types of materials, and advantageously can be dried in air without the need for high temperature treatment, thus allowing the coating of materials that do not support high temperatures, such as many plastic materials.

Preferably, the particles of inorganic semi-conductor exhibit an average diameter smaller than 100 nm. Advantageously, the weight of the inorganic semi-conductor particles is two to twenty times less than the weight of both the diluent and binder, preferably 3 to 15 times less.

The polymeric gel containing the particles of semi-conductor is applied onto an inner surface of the tubular wall portion 5 in order to use its photocatalytic properties to clean and deodorize air. Preferably, the thickness of the gel layer is less than 1 mm on the surface of the tubular wall portion.

As illustrated in figure 2B the blades 8 may be inclined at an angle of inclination β relative to the inner surface of the tubular portion 5. The angle of incline β of the blades relative to the inner surface of the tubular wall portion may be between 40° to 140°, preferably between 55° and 110°, more preferably between 55° and 75°, for instance around 60°.

As illustrated in figure 2B the blades 8 may be inclined at an angle of inclination α relative to the central axis A of the tubular portion. The angle of incline α of the blades relative to the central axis A may be between 0° to 90°, preferably between 45° to 85°, for instance around 60°.

Having one or more blades inclined at an angle α, preferably around 60°, relative to the central axis of the conduit and/or inclined at an angle β, of preferably around 60°, relative to the inner surface of the tubular wall portion enables optimal exposure of the surface of the blades coated with photocatalytic material to UV light irradiation from a UV light source placed in the conduit. The thus inclined blades provide an optimised the flow-path of the air passing through the catalyser device. The angle of incline of the blades allows the turbulance created by the blades to be optimised to ensure that the air is well mixed and comes into contact with the photo-catalytic material. Further by having the blades inclined towards the inner surface of the tubular wall portion air flowing through the catalyser device is forced along the surface of the blades ensuring good contact of the air with the photcatalytic material.

The ultraviolet light source 4, is preferably positioned substantially along the central axis of the catalyser tube. Advantageously the ultraviolet light source generates light having a wavelength between 180 and 400 nm, preferably between 240 and 420 nm. Preferably the ultraviolet light source generates light having a wavelength of 254nm. Use of light having a wavelength of 254nm advantageously allows the production of ozone to be avoided. The ultraviolet light source 4 may be one or more UV lamps. For instance, it can be a low or medium pressure mercury lamp, an incandescent lamp or a fluorescent lamp. It may have a cylindrical shape, a bulb shape or any other shape.

The distance between the inner irradiated surface of the tubular portion is advantageously between 0.5 cm to 5 cm preferably between 1 cm to 3cm from the ultraviolet light source in order to optimize the overall effectiveness of the cleaning and deodorizing activity of the apparatus by optimizing the balance between the intensity of the photocatalytic activity of the gel and the direct germicidal action of the UV light with the surface area of the photocatalytic material in contact with the air.

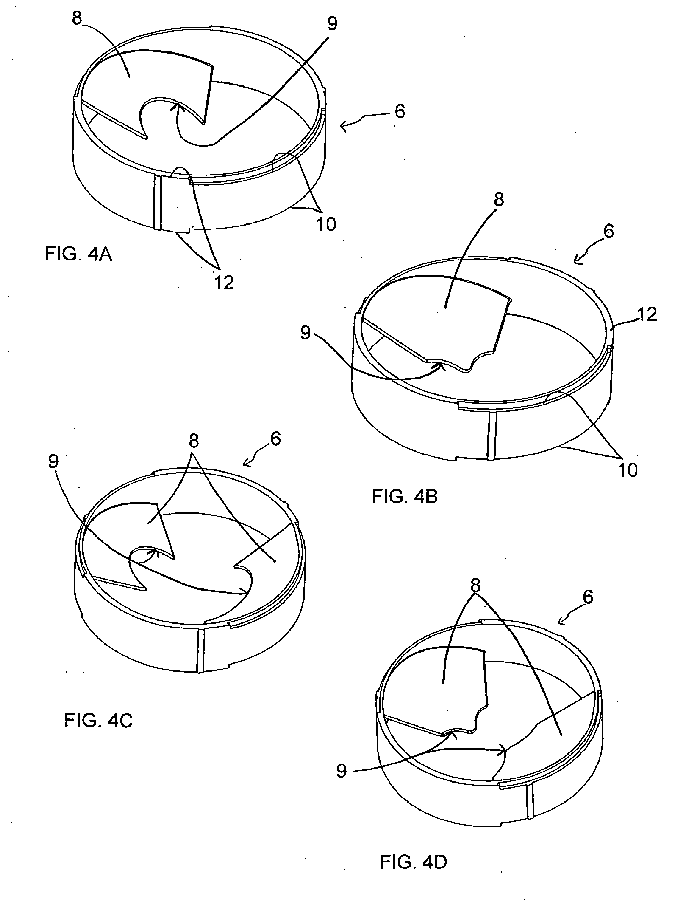

Figures 4 A, B, C and D illustrate embodiments of annular elements according to an embodiment of the invention. The annular elements 6 may advantageously have one blade portion as illustrated in figures 4A and 4B or two blade portions as illustrated in figure 4C and 4D. The inner edge 9 of the blade is advantageously contoured to provide a complementary fit with the contour of an outside surface of the ultraviolet light source 4. In the embodiments illustrated in figure 4 the inner edges of the blades are contoured to fit complementarily with a double tube UV light source.

The annular sections may be releasably connected to from a tube with a releasable locking means, or may be held together, e.g by the housing, without any locking means. As a releasable locking means, for instance a clipping mechanism may be used. A clipping mechanism may, for instance, comprise a series of complementary grooves and lips 10, 12. Alternatively in another embodiment (not illustrated) the annular elements may be rigidly fixed together by bonding, sealing, welding, or other fixing means.

The removable catalyser device 3 is positioned in the housing so that its surface coated with the photocatalytic layer receives as much irradiating light rays as possible from the ultraviolet light source 4. The removable catalyser device 3 may be easily accessed by taking off the housing 2, and subsequently removed and replaced by another identical removable catalyser device. The air conduit may have a simple cylindrical shape, or may take different forms according to different shapes or different uses of the apparatus according to the present invention.

The apparatus may further comprise an airflow system, and blows the air through the catalyser device 3, in which the ultraviolet light source 4 is positioned. The airflow system may comprise a blade fan driven by an electric motor. The airflow system may further comprise a filter at its inlet to remove insects, large particles and dust.

The apparatus 1 uses two physico-chemical processes for cleaning and deodorizing air and two complementary technologies: the germicidal action of ultraviolet light rays and the mineralization via photocatalysis. Altogether, those two technologies actively fight against pathogenic and/or allergenic micro-organisms as well as against volatile organic compounds originating from the industrial pollution. The apparatus 1 allows, by combining the afore-mentioned technologies, to efficiently purify ambient air by destroying micro-organisms, including bacteria, protozoa and particularly spores, as well as volatile organic compounds and odour compounds. The apparatus may be used as a home appliance or for professional purpose as well, according to its size.

After a predetermined time period of use, depending on the use of the apparatus, e.g., every twelve months, the catalyser device 3 may be removed and replaced by another identical catalyser device.

Thus, the present invention is effective for cleaning and deodorising air and is easy to manufacture, use and maintain.

An apparatus for cleaning and deodorising large volumes of air could comprise a plurality of catalyser devices and corresponding ultraviolet light sources grouped together in a single housing or unit. The catalyser devices could, for example, have a hexagonal profile to enable grouping together in a sort of "honeycomb" structure.

## Claims

1. A catalyser device for cleaning and deodorising air, comprising an air conduit and a photocatalytic material in the conduit, wherein the air conduit comprises a tubular wall portion and a plurality of spaced apart blades extending from an inner surface of the tubular portion towards a central axis of the conduit.

2. A catalyser device according to claim 1 wherein the photocatalytic material is coated on at least a portion of the inner surface of the tubular portion and the blades.

3. A catalyser device according to claim 1 or 2 wherein the tubular portion comprises a plurality of annular elements.

4. A catalyser device according to any one of claims 1 to 3 wherein one or more of said plurality of blades are inclined relative to the central axis of the conduit and/or inclined relative to the inner surface of the tubular portion.

5. A catalyser device according to claim 4 wherein the one or more inclined blades have an angle of incline relative to the central axis in the range of 45° to 85°.

6. A catalyser device according to claim 4 wherein the one or more inclined blades have an angle of incline relative to the inner surface of the tubular portion in the range of 55° and 75°.

7. A catalyser device according to any one of claims 1 to 6 wherein the plurality of blades are in a substantially helical arrangement.

8. A catalyser device according to any one of claims 1 to 7, wherein the tubular portion is made of a plastic.

9. A catalyser device according to any one of claims 1 to 8 wherein the tubular portion has a generally cylindrical shape.

10. A catalyser tube according to any one of claims 1 to 9 where the photocatalytic material comprises an inorganic binder including ultraviolet light permeable polymeric molecules, a polar diluent, and particles of an inorganic semi-conductor, the weight of the inorganic semi-conductor particles being two to twenty times less than the weight of both the diluent and binder.

11. A catalyser tube according to claim 10 wherein the polymeric molecules include acrylic molecules.

12. Apparatus for cleaning and deodorizing air, comprising a housing, a catalyser device comprising an air conduit and a photocatalytic material in the conduit, an ultraviolet light source received in the air conduit for irradiating an inner surface thereof, and an airflow system for propelling or drawing air through the catalyser device, wherein said air conduit and comprises a tubular wall portion and a plurality of spaced apart blades extending from an inner surface of the tubular wall portion towards a central axis of the conduit.

13. Apparatus according to claim 12 wherein the catalyser device is removably mounted in the housing.

14. Apparatus according to claim 12 or 13 wherein the catalyser devise has the properties of the device according to any one of claims 1 to 11.

15. Apparatus according to any on of claims 12 to 14 wherein the ultraviolet light source is in the form of one or more UV light tubes removably mounted substantially along the central axis of the air conduit.

16. Apparatus according to any one of claims 12 to 15 wherein the inner surface of the tubular wall portion is positioned at a distance between one to three centimetres from the ultraviolet light source.

17. Apparatus according to claim 15 or 16 wherein a radially interior edge said plurality of blades has a form complimentary with the form of an outside surface of the UV light tube.

18. Apparatus according to any one of claims 12 to 17 wherein the airflow system comprises a blade fan driven with an electric motor.
